# EUROPEAN PATENT SPECIFICATION

(11) **EP 2 288 267 B1**
(45) Date of publication and mention of the grant of the patent: **29.02.2012**
(21) Application number: 09753725.2
(22) Date of filing: 07.04.2009
(51) Int. Cl.: A23K 1/16, A23K 1/165, A23K 1/18

(54) **AN ENZYMATIC PRE-MIXTURE AGAINST GRAM NEGATIVE BACTERIA COLONIZATION IN THE ANIMAL INTESTINAL TRACT**
ENZYMATISCHE VORMISCHUNG GEGEN GRAM-NEGATIVE BAKTERIENKOLONISATION IM DARMTRAKT VON TIEREN
PRÉ-MÉLANGE ENZYMATIQUE CONTRE LA COLONISATION PAR DES BACTÉRIES GRAM NÉGATIVES DANS LE TRACTUS INTESTINAL D'ANIMAUX

(30) Priority: 14.04.2008 EP 08154484
(43) Date of publication of application: 02.03.2011
(73) Proprietor: Institut de Recerca i Tecnologia Agroalimentàries, 08007 Barcelona (ES); Industrial Técnica Pecuaria S.A., 08011 Barcelona (ES)
(72) Inventor: BRUFAU DE BARBERÀ, Joaquím, E-43201 Reus (Tarragona) (ES); PÉREZ VENDRELL, Ana María, E-43382 Maspujols (Tarragona) (ES); BADIOLA SANZ, Ignasi, E-08860 Castelldefels (Barcelona) (ES); PERIS MIRAS, Silvia, E-08970 Sant Joan Despí (Barcelona) (ES)
(74) Representative: ZBM Patents
(86) International application number: PCT/EP2009/054172
(87) International publication number: WO 2009/144070

(56) References cited:
- EP-A- 1 881 067
- EP-A2- 1 892 292
- WO-A-2007/002690
- US-A1- 2004 151 804
- US-A1- 2006 228 448
- DATABASE WPI Week 20043 Thomson Scientific, London, GB; AN 2004-035396 XP002498757 & WO 03/101219 A (INST RICERCA I TECNOLOGIA AGROALIMENTAIR) 11 December 2003 (2003-12-11) cited in the application
- DATABASE WPI Week 200159 Thomson Scientific, London, GB; AN 2001-536641 XP002498758 & WO 01/62951 A (FUJI OIL CO LTD) 30 August 2001 (2001-08-30)
- DATABASE WPI Week 199625 Thomson Scientific, London, GB; AN 1996-246903 XP002498759 & JP 08 099884 A (TAIYO KAGAKU KK) 16 April 1996 (1996-04-16)
- DATABASE WPI Week 200232 Thomson Scientific, London, GB; AN 2002-275205 XP002498760 & JP 2002 012505 A (DAICEL CHEM IND LTD) 15 January 2002 (2002-01-15)
- YOO J S ET AL: "The effects of environment-friendly diets on the growth performance, nutrient digestibility, fecal excretion, nitrogen excretion and emission gases in manure for growing pigs" JOURNAL OF ANIMAL SCIENCE AND TECHNOLOGY, vol. 49, no. 4, August 2007 (2007-08), pages 491-500, XP009106838 ISSN: 1598-9429

## Description

The present invention relates to a pre-mixture for animal feeding which is effective in the reduction or prevention of pathogenic Gram negative bacteria in the animal intestinal tract.

### BACKGROUND ART

Poultry and meat are frequent sources of food borne disease organisms. The pathogens associated with poultry products include *Salmonella* spp., *Listeria monocytogenes, Escherichia coli* and *Campylobacter jejuni.* These microorganisms are introduced into poultry and animal flocks during raising and onto carcasses during processing. These bacteria colonize the mucus covering gut surfaces and can also be found in deep mucus of intestinal crypts.

The infection of *Salmonella enteritidis* (SE) in poultry is one of the most serious problems in the poultry industry because of related outbreaks of food poisoning. Much effort has been made to inhibit *Salmonella* spp. infection in poultry. The inhibition of *Salmonella* spp. with antibiotics does not seem to be practical because of the occurrence of resistant bacterial strains and the residual toxicity of antibiotics in poultry products.

Recently vaccination has become a common approach, but it has several limitations. Vaccine therapy cannot distinguish whether the origin of the antibody derives from the vaccine or from a bacterial pathogen when *Salmonella* serology is done. Thus, the period of vaccination must be strictly programmed and is restricted to a specific interval of time. It has been reported that some kinds of carbohydrates, yeast, and, especially, the mannose residue are effective in preventing the *Salmonella* spp. colonization.

Other recent approach is based on the use of certain monomeric and dimeric sugars in the poultry diet to reduce the levels of Salmonella organisms that colonize their gut. As previously indicated, sugar receptors on the surfaces of intestinal epithelial cells apparently serve as receptors for the binding of some bacterial pathogens. The interaction between the Gram-negative bacterial pili and these receptors can be blocked by certain simple sugars in the animal feed.

In the PCT Publication number WO03101219 it is described a pre-mixture for use in animal feeding. In this application a pre-mixture comprising locust bean gum, a liquid hydrolase preparation and sepiolite (which is an acceptable additive for animal feeding) is described. This mixture was used against Salmonella's colonization in chicks.

Despite the efforts made in the prior art, the research of new compositions more effective in the prevention or inhibition of colonization of Gram negative bacteria (such as Salmonella spp.) is still an active field.

### SUMMARY OF THE INVENTION

Inventors have found that a pre-mixture of a polymer with a content in mannose comprised between 10% and 100%; a β-mannanase enzyme; and an anti-binding with an specific absorptive capacity and bulk density is effective against Gram negative bacteria colonization in the animal intestinal tract. Particularly, this pre-mixture has a high fluidity, implying a better diffusion through the intestinal tract. The main advantages of this high fluidity and diffusion is that the efficacy of the pre-mixture is increased and, therefore, a low inclusion dose (i.e., amount of the mixture into the feed animal) for achieving the desired effect (i.e., to avoid bacterial colonization) is needed. As it is shown below, a small amount of the mixture of the invention is enough to achieve the desired effect, which means that there is a small raise of the total of the fodder that must be administered. This is of great importance because when the farm animal has to be nourished, a fix amount of fodder is administered. If the mixture of the invention had to be supplied in a high amount, the amount of fodder should be reduced and, therefore, the diet of the animal would not be appropriate for its growth. In this case additional nutrients should be added to the fodder to help to the appropriate growth of the animal.

From the teachings of WO03101219 it was known that a mixture comprising sepiolite as technological acceptable additive, together with a liquid hydrolase preparation, and a natural gum, has the ability of inhibiting Salmonella's colonization. In the examples of said application, the mixture amounts used are of 2.5% and 5% by weight over the total fodder weight, i.e., from 5 to 10-fold higher than the one used in the examples included below for inhibiting the bacteria colonization.

Advantageously, as the mixture of the invention is effective at a low amount (for instance, in Tables 2 and 3 the amount is 0.5% by weigth/total fodder), it is practically maintained the amount of fodder, maintaining the levels of nutrients needed to the appropriate growth of the animal and, therefore, being not needed the addition of other components.

On the other hand, it is relevant that when the pre-mixture of the invention is administered, the conversion index is maintained when compared with a control. A value of the conversion index significantly equal to the control group is indicative of a good transformation (the food administered to the animal is transformed in meat/eggs). A different value significantly higher is indicative of an inappropriate transformation (a huge amount of food must be administered to the animal in order to achieve the desired amount of meat/eggs). As it is illustrated below (Table 1, line T-3), the pre-mixture described in WO03101219 has a conversion index significantly different from the conversion index of the control group. Advantageously, when the pre-mixture of the invention is administered (Table 2, lines T-2 and T-3) there no significant differences with the control group.

Thus, in a first aspect the present invention refers to a pre-mixture for animal feeding comprising: (a) a polymer or a mixture of polymers, having a content in mannose comprised between 10% and 100%; (b) β-mannanase enzyme; and (c) an anti-binding agent; wherein the anti-binding agent has an absorptive capacity higher than 20%, and a bulk density which is comprised between 0.10 and 2.70 g/cm³.

Surprisingly, the use of an anti-binding agent having such properties (i.e., the absorptive capacity and bulk density) instead of sepiolite, improves the efficacy in the inhibition of colonization. This improvement makes possible the use of the pre-mixture of the invention in an amount about at least 5 fold lower than the amount needed in WO03101219 to achieve the same effect (as it has been explained above).

Without being bound to the theory, it is believed that using an anti-binding agent according to the first aspect of the invention, the enzyme diffuses better in the pre-mixture, being more effective when it has to hydrolyze its substrate (i.e., the polymer).

The diffusion improvement also affects to the resulting product (the polymer hydrolyzed by the β-mannanase). Thus, the resulting product, owing to the presence of the anti-binding agent, diffuses better into the intestinal tract, being its efficacy in the inhibition of the bacterial colonization improved.

In a second aspect, the present invention refers to an animal feed composition comprising the pre-mixture as defined according to the first aspect of the invention.

In a third aspect, the present invention refers to the use of a pre-mixture according to the first aspect of the invention, for the preparation of an animal feed composition for the inhibition of Gram negative bacteria colonization in the animal intestinal tract. This aspect may be formulated as a method of treatment of an animal, suffering from or being susceptible of suffering from a Gram negative bacteria colonization by administering the pre-mixture of the present invention as defined above.

It is also part of the invention a pre-mixture as defined above for use as inhibitor of Gram negative bacteria colonization in the animal intestinal tract.

### DETAILED DESCRIPTION OF THE INVENTION

In the present invention, the expression "a polymer with a content in mannose comprised between 10% and 100%" encompasses homopolysaccharides consisting exclusively of mannose units (i.e., a polysaccharide with a content of 100% in mannose) and heteropolysaccharides comprising mannose and other sugar units such as glucose, galactose, α-galactose derivative, galactomannans, galactosamine, fucose, and arabinose, for instance. Polymers according to the present invention includes, but are not limited to: guar gum, xanthan gum, pectin, gum tragacanth, gum arabic, cassia gum, palm kernel meal, algin, carrageenan, locust bean gum, locust bean seed, and mixtures thereof.

In one embodiment of the first aspect of the invention, the polymer has a content in mannose comprised between 10% and 60%.

In the present invention, the term "anti-binding agent" encompasses any substance which reduces or avoids the adhesion between the compound particles resulting from the β-mannanase activity.

In a preferred embodiment of the first aspect of the invention, the anti-binding agent has an absorptive capacity comprised between 60 and 70% and a bulk density comprised between 0.10 and 0.30 g/cm³.

The absorptive capacity of the anti-binding agent is determined using the following protocol:
1.- Tare a 50 mL volumetric flask over the scale.
2.- Add 50 g of the anti-binding agent to be determined.
3.- With the graduated cylinder add gradually deionized water and stir with the glass bar until its total absorption by the anti-binding agent.
4.- Repeat this operation (point 3) until the anti-binding agent will not be able to absorb more liquid.
5.- The analysis must be performed by duplicate.
6.- The following equation is applied:
   Absorption capacity = [g deionized water / (g deionized water + g anti-binding agent)] * 100.

The bulk density of the anti-binding agent is determined using the following protocol:
1.-Tare a 50 ml graduated cylinder over the semi-analytical balance (0.01 g accuracy).
2.-Fill the graduated cylinder to the line with the anti-binding agent. Don't knock the graduated cylinder in order to avoid incorrect results.
3.-Weigh the filled cylinder.
4.-Make the determination twice.
5.- Bulk density = weight (g) / 50 ml = g/ml. (The final results is the mean of both determinations).

When the pre-mixture of the invention is prepared, it is preferably obtained as a homogenous solid mixture. In order to obtain the homogeneous solid mixture, the enzyme can be in solid form, e.g., powder.

The fact that the enzyme is in a solid form, makes easier its handling and improves its diffusion through the intestinal tract. In WO03101219 the sepiolite additive was used as vehicle in order to "solidify" the enzyme, which was initially in the form of liquid preparation. In the present invention, the use of an anti-binding agent with the properties pointed out above avoids the appearance of lumps in the pre-mixture, which would difficult the diffusion of the mixture into the intestinal tract and, hence worsening its effectiveness in the inhibition of the bacteria colonization.

In still another preferred embodiment of the first aspect of the invention, the ratio of the enzyme is comprised between 5 and 40 enzymatic activity units per gram of polymer. The enzymatic activity units are determined for the b-mannanase enzyme as quantity of enzyme that releases 1 micromol of mannosa per minute from carob galactomannan at 40°C and pH= 4.

In another embodiment of the first aspect of the invention, the polymer is selected from the group consisting of: guar gum, xanthan gum, pectin, gum tragacanth, gum arabic, algin, carrageenan, locust bean gum, locust bean seed, cassia gum, palm kernel meal, and mixtures thereof.

In the present invention the "β-mannanase enzyme" comprised in the pre-mixture of the invention can be of natural or synthetic origin. By "synthetic origin" it is encompassed any form of the enzyme obtained by engineering techniques (e.g., recombination).

In a preferred embodiment of the first aspect of the invention the β-mannanase enzyme is E.C. 3.2.1.78 according to the nomenclature of the International Union of Biochemistry.

In another preferred embodiment of the first aspect of the invention, the amount of anti-binding agent is comprised between 0.5 and 4% by weight with the respect the total weight of the pre-mixture.

Illustrative non-limitative examples of anti-binding agents are: precipitated and dried acid silicic (E551 a), colloidal silica (E551 b), vermiculite (E561), Kieselgur (E551 c), synthetic calcium silicate (E552), synthetic sodium and aluminium silicate (E554), synthetic calcium aluminates (E598), clinoptilolite from volcanic origin (E567). The "E" number, for instance "E551 a", corresponds to the additive number authorized in the European Union. In a preferred embodiment of the first aspect of the invention, the anti-binding agent is selected from the group consisting of: precipitated and dried acid silicic (E551a), colloidal silica (E551 b), and vermiculite (E561).

In a preferred embodiment of the second aspect of the invention, the pre-mixture is comprised between 0.05 and 1.5 % by weight of the total composition.

As it has been mentioned throughout the description, the pre-mixture of the present invention is useful in the effective inhibition of the colonization of Gram negative bacteria. The term "Gram negative bacteria" includes, but it is not limited to, bacteria exhibiting fimbria, such as *Enterobacteriaceae*, among others. In a preferred embodiment of the third aspect of the invention, the Gram negative bacteria is *Salmonella.*

Throughout the description and claims the word "comprise" and variations of the word, such as "comprising", is not intended to exclude other technical features, additives, components, or steps.

The following examples are provided by way of illustration, and is not intended to be limiting of the present invention.

### EXAMPLES

### Example 1: Preparation of a pre-mixture according to the present invention in a proportion of 0.5°l° in weight in relation to the diet for the animal

Per ton of feed the premix is prepared as follows:
(i) Locust bean gum: 4.482,5 g
(ii) Beta-mananase: 21,5 g
(iii) Locust bean seed: 446 g
(iv) Silicic acid: 50 g

To obtain the premix of this finding: (1) components (iv) and (iii) have to be mixed on a first stage. On a second phase: (2) 1/3 of (i) has to be mixed with the totality of (ii). After the homogeneous mix, add the rest of (i) to this last premix. Finally, mix homogeneously (1) and (2).

### Example 2. Trial with Leghorn chickens consuming feed supplemented with different products of the state of the art for 28 days

Chickens were allocated in cages, at the rate of 5 animals/cage, in the experimental farm of the Animal Nutrition Department of IRTA. Diet was based on corn-soyabean in mash form. Application of the premix was always by substitution of corn.

Chickens were challenged with *Salmonella enteritidis* marked with nalidixic acid. Efficacy of the application of each one of the products was assessed by means of a determination of *Salmonella* presence in chickens' caecum. Chicken's were challenged at one day old, when they arrived to the experimental farm, by inoculating *Salmonella enteritidis* (S-2146/c/03 Tn 5¹²), strains resisting 20 µg/ml nalidixic acid and 12 µg/ml ClHg. Strains were originated from the L.S.A. collection, Barcelona.

During the 28 days of the experiment data from feed consumption and initial and final body weights were collected. With those data the following parameters were calculated: Daily Body Weight Gain (DBWG) (g/d), Daily Feed Intake (DFI) (g/d) and Feed Conversion Index (FCI) (Daily Feed Intake / Daily Body Weight Gain). a,b,c: different letters in the same column mean significant differences.

**Table 1**

| **Treatment** | **Added product** | **DBWG¹** | **DFI²** | **FCI³** | **Infection/ 15 days** | |
|---|---|---|---|---|---|---|
| T-1 | Control (no added product) | 8.9 (a) 8.9 (a) | 18.2 (a) | 2.04 (c) | 12/12 | 100% |
| T-2 | Locust bean gum 2.5% | 6.8 (b) 6.8 (b) | 17.2(a) | 2.52 (a) | 6/12 | 50% |
| T-3 | Locust bean gum 5 % + sepiolite + β-mananase (WO03101219) | 6.9 (b) | 17.0(a) | 2.46 (ba) | 4/12 | 25% |

Using the pre-mixture described in WO030101219, a reduction in the infection is achieved. However, the index of conversion is significantly higher compared with the control and it is observed a decrease in the body weight gain.

### Example 3: Experimental results using the pre-mixture of the invention

Hens were allocated in cages, at the rate of 5 animals/cage, in the experimental farm of the Animal Nutrition Department of IRTA. Diet was based on corn-soyabean in mash form. Application of the premix prepared as explained in Example 1 was always by substitution of corn.

Each 4 weeks data from feed consumption and changes in body weight were recorded. With those data the Feed Conversion Index (Daily Feed Intake / Daily Body Weight Gain) was calculated. Results are shown in Table 2.

On day 27 of treatment, all hens were orally challenged with *Salmonella enteritidis.* The strains used were a mixture of GN825 and GN1063, which had been selected from the most invasive in previous trials. In two of the treatments, control and New product at 0.5%, every fourth week (at the end of each 4 week period), 8 animals per treatment were euthanized and ovarios and caecum examined for *Salmonella* presence/absence. *Salmonella enteritidis* study was conducted in accordance with the modified ISO standard. Briefly: tissue samples were collected in sterile plastic containers with 225 ml of buffered peptone water; incubation; addition of 3 drops of the pre-enrichment broth in a semi-solid media Rappaport-Vassiliadis and incubation of this media. Putative *Salmonella* white colonies were transferred on XLT4 agar plates to confirm the presence/absence of *Salmonella.* Results are shown in Table 3.

**Table 2**

| **Treatment** | **Added product** | **Body Weight Change (g)** | **DFI¹ (g)** | **FCI²** |
|---|---|---|---|---|
| T-1 | Control (no added product) | 48 | 106 | 2.33 |
| T-2 | New product 0.5% | 45 | 104 | 2.39 |
| T-3 | New product 5% | 44 | 103 | 2.41 |

From the results obtained with the pre-mixture according to the present invention, the conversion index is substantially maintained compared with the control group and the body weight change when the pre-mixture is administered to the infected animals is practically maintained when compared with the control group.

**Table 3. Results of microbiological analysis from caecum and ovary at different time periods after inoculation (% of birds with presence of Salmonella).**

| Treatment | Added product | Before inoculation | Infection / 30 days | Infection/ 90 days | Infection / 120 days | Infection / 150 days |
|---|---|---|---|---|---|---|
| T-1 | Control (no product added) | 0,0 | 25,0^{a} | 25,0^{a} | 12,5^{a} | 12,5^{a} |
| T-2 | Product of the invention 0.5% | 0,0 | 6,3^{b} | 6,3^{b} | 0,0^{b} | 0,0^{b} |

## Claims

1. A pre-mixture for animal feeding comprising:
(a) a polymer or a mixture of polymers with a content in mannose comprised between 10% and 100%;
(b) β-mannanase enzyme; and
(c) an anti-binding agent;
wherein the anti-binding agent has an absorptive capacity higher than 20%, and a bulk density which is comprised between 0.10 and 2.70 g/cm³.

2. The pre-mixture according to claim 1, wherein the polymer has a content in mannose comprised between 10% and 60%.

3. The pre-mixture according to any of the previous claims, wherein the anti-binding agent has an absorptive capacity comprised between 60 and 70% and a bulk density comprised between 0.10 and 0.30 g/cm³.

4. The pre-mixture according to any of the previous claims, which is in a homogeneous solid form.

5. The pre-mixture according to any of the preceding claims, wherein the ratio of the enzyme is comprised between 5 and 40 enzymatic activity units per gram of polymer.

6. The pre-mixture according to any of the preceding claims, wherein the amount of anti-binding agent is comprised between 0.5 and 4% by weight with the respect the total weight of the pre-mixture.

7. The pre-mixture according to any of the preceding claims, wherein the polymer is selected from the group consisting of: guar gum, xanthan gum, pectin, gum tragacanth, gum arabic, algin, carrageenan, locust bean gum, locust bean seed, cassia gum, palm kernel meal, and mixtures thereof.

8. The pre-mixture according to any of the preceding claims, wherein the 13-mannanase enzyme is E.C. 3.2.1.78 according to the nomenclature of the International Union of Biochemistry.

9. The pre-mixture according to any of the preceding claims, wherein the anti-binding agent is selected from the group consisting of: silicic acid, colloidal silica, and vermiculite.

10. An animal feed composition comprising the pre-mixture as defined in any of the claims 1-9.

11. A pre-mixture as defined in any of the claims 1-9, for use as inhibitor of Gram negative bacteria colonization in the animal intestinal tract.

12. Use of a pre-mixture as defined in any of the claims 1-9, for the preparation of an animal feed composition for the inhibition of Gram negative bacteria colonization in the animal intestinal tract.

13. The pre-mixture according to claim 12, wherein the Gram negative bacterium is of *Salmonella* spp.

## Patentansprüche

1. Vormischung zur Tierfütterung bestehend aus:
(a) ein Polymer oder eine Polymermischung mit einem Gehalt an Mannose zwischen 10% und 100%;
(b) Enzyme β-Mannanase; und
(c) ein Antibindemittel;
wobei das Antibindemittel eine Absorptionsfähigkeit größer als 20% hat und eine Schüttdichte hat, deren Wert zwischen 0,10 und 2,70 g/cm³ liegt.

2. Vormischung nach Anspruch 1, wobei das Polymer einen Gehalt an Mannose zwischen 10% und 60% hat.

3. Vormischung nach einem der vorangehenden Ansprüche, wobei das Antibindemittel eine Absorptionsfähigkeit hat, deren Wert zwischen 60 und 70% liegt und eine Schüttdichte hat, deren Wert zwischen 0,10 und 0,30 g/cm³ liegt.

4. Vormischung nach einem der vorangehenden Ansprüche, die in einer homogenen festen Form vorliegt.

5. Vormischung nach einem der vorangehenden Ansprüche, wobei das Verhältnis von Enzyme zwischen 5 und 40 Einheiten der Enzymaktivität pro Polymergramm liegt.

6. Vormischung nach einem der vorangehenden Ansprüche, wobei der Gehalt an Antibindemittel zwischen 0,5 und 4% Gewichtsprozent bezüglich des gesamten Gewichts der Vormischung liegt.

7. Vormischung nach einem der vorangehenden Ansprüche, wobei das Polymer ausgewählt aus der Gruppe ist, bestehend aus: Guargummi, Xanthan, Pektin, Tragantgummi, Gummiarabikum, Algin, Carrageen, Johannisbrotkernmehl, Johannisbrotkern, Kassiagummi, Palmkernmehl und Mischungen daraus.

8. Vormischung nach einem der vorangehenden Ansprüche, wobei das Enzym β-Mannase nach der Nomenklatur der International Union of Biochemistry bezeichnet als E.C. 3.2.1.78 wird.

9. Vormischung nach einem der vorangehenden Ansprüche, wobei das Antibindemittel ausgewählt aus der Gruppe ist, bestehend aus: Kieselsäure, Kolloidkieselerde und Vermiculit.

10. Zusammensetzung zur Tierfütterung, die die Vormischung nach einem der Ansprüche 1-9 umfasst.

11. Vormischung nach einem der Ansprüche 1-9 zur Verwendung als Inhibitor der Besiedlung durch Gram-negative Bakterien im Verdauungstrakt von Tieren.

12. Verwendung einer Vormischung nach einem der Ansprüche 1-9 zur Herstellung einer Zusammensetzung zur Tierfütterung zur Inhibition der Besiedlung durch Gram-negative Bakterien im Verdauungstrakt von Tieren.

13. Vormischung nach Anspruch 12, wobei die Gram-negative Bakterie *Salmonella* spp ist.

## Revendications

1. Pré-mélange destinée à l'alimentation animale comprenant :
(a) un polymère ou mélange de polymères avec une teneur en mannose comprise entre10% et 100%;
(b) de l'enzyme β-mannanase ; et
(c) an agent antiagglomérant ;
dans laquelle l'agent antiagglomérant a une capacité d'absorption supérieure à 20 %, et une densité apparente comprise entre 0,10 et 2,70 g/cm³.

2. Pré-mélange selon la revendication 1, dans laquelle le polymère a une teneur en mannose comprise entre 10 % et 60 %.

3. Pré-mélange selon l'une quelconque des revendications précédentes, dans laquelle l'agent antiagglomérant a une capacité d'absorption comprise entre 60 et 70 % et une densité apparente comprise entre 0,10 et 0,30 g/cm³.

4. Pré-mélange selon l'une quelconque des revendications précédentes, étant sous une forme solide homogène.

5. Pré-mélange selon l'une quelconque des revendications précédentes, dans laquelle le rapport d'enzyme est compris entre 5 et 40 unités d'activité enzymatique per gram de polymère.

6. Pré-mélange selon l'une quelconque des revendications précédentes, dans laquelle la quantité d'agent antiagglomérant est comprise entre 0,5 et 4 % en poids par rapport au poids total de la pré-mélange.

7. Pré-mélange selon l'une quelconque des revendications précédentes, dans laquelle le polymère est choisi dans le groupe constitué de : gomme de guar, gomme xanthane, pectine, gomme adragante, gomme arabique, algine, carraghénane, farine de grains de caroube, graines de caroube, gomme de casse, farine de palmiste, et leurs mélanges.

8. Pré-mélange selon l'une quelconque des revendications précédentes, dans laquelle l'enzyme β-mannanase est E.C. 3.2.1.78 selon la nomenclature de l'Union international de biochimie.

9. Pré-mélange selon l'une quelconque des revendications précédentes, dans laquelle l'agent antiagglomérant est choisi dans le groupe constitué de : acide silicique, silice colloïdale, et vermiculite.

10. Composition alimentaire animale comprenant la pré-mélange telle que définie dans l'une quelconque des revendications 1 à 9.

11. Pré-mélange telle que définie dans l'une quelconque des revendications 1 à 9, destinée à être utilisée en tant qu'un inhibiteur de la colonisation de bactéries à Gram négatif dans le tractus intestinal des animaux.

12. Utilisation d'une pré-mélange telle que définie dans l'une quelconque des revendications 1 à 9 pour la préparation d'une composition alimentaire animale pour l'inhibition de la colonisation de bactéries à Gram négatif dans le tractus intestinal des animaux.

13. Pré-mélange selon la revendication 12, dans laquelle la bactérie à Gram négatif est *Salmonella* spp.
